# EUROPEAN PATENT SPECIFICATION

(11) **EP 4 503 226 B1**
(45) Date of publication and mention of the grant of the patent: **14.05.2025**
(21) Application number: 24191920.8
(22) Date of filing: 30.07.2024
(51) Int. Cl.: H01M 10/052, H01M 10/0567, H01M 10/0569

(54) **ELECTROLYTE FOR LITHIUM SECONDARY BATTERY AND LITHIUM SECONDARY BATTERY INCLUDING THE SAME**
ELEKTROLYT FÜR LITHIUMSEKUNDÄRBATTERIE UND LITHIUMSEKUNDÄRBATTERIE DAMIT
ÉLECTROLYTE POUR BATTERIE SECONDAIRE AU LITHIUM ET BATTERIE SECONDAIRE AU LITHIUM LE COMPRENANT

(30) Priority: 31.07.2023 KR 20230100067
(43) Date of publication of application: 05.02.2025
(73) Proprietor: SK On Co., Ltd., Seoul 03161 (KR); SK Innovation Co., Ltd., Seoul 03188 (KR)
(72) Inventor: SHIM, Yu Na, 34124 Daejeon (KR); JEON, Hyun Ji, 34124 Daejeon (KR)
(74) Representative: Stolmár & Partner Patentanwälte PartG mbB

(56) References cited:
- CN-A- 106 159 323
- US-A1- 2023 101 481

## Description

### [BACKGROUND OF THE INVENTION]

### 1. Field of the Invention

The present disclosure relates to an electrolyte for a lithium secondary battery and a lithium secondary battery including the same, and more specifically, to an electrolyte for a lithium secondary battery which includes a solvent and electrolyte salts, and a lithium secondary battery including the electrolyte.

### 2. Description of the Related Art

A secondary battery is a battery which can be repeatedly charged and discharged, and has been widely applied to portable electronic devices such as a mobile phone, a laptop computer, etc. as a power source thereof. A lithium secondary battery has a high operating voltage and a high energy density per unit weight, and is advantageous in terms of a charging speed and light weight. In this regard, the lithium secondary battery has been actively developed and applied to various industrial fields.

For example, the lithium secondary battery may include: an electrode assembly including a cathode, an anode, and a separation membrane interposed between the cathode and the anode; and an electrolyte in which the electrode assembly is impregnated in a case. A lithium secondary battery having longer lifespan, high capacity, and operational stability is required as the application range thereof is expanded.

As an active material for the cathode of the lithium secondary battery, a lithium metal oxide may be used. Examples of the lithium metal oxide may include a nickel-based lithium metal oxide. However, output and capacity of the secondary battery may be decreased due to surface damage of the cathode active material caused by repeated charging and discharging in a high temperature environment, and a side reaction between the cathode active material and the electrolyte may occur.

Accordingly, a development of a lithium secondary battery which provides uniform output and capacity even during repeated charging and discharging is required. CN 106 159 323 A discloses an electrolyte for a lithium secondary battery comprising: an additive which is EDTA, an organic solvent and a lithium salt.

US 2023/101481 A1 discloses an electrolyte for a lithium secondary battery comprising: an additive which is an amino heterocyclic compound, an organic solvent and a lithium salt.

### [SUMMARY OF THE INVENTION]

An object of the present disclosure is to provide an electrolyte for a lithium secondary battery having improved high-temperature properties.

Another object of the present disclosure is to provide a lithium secondary battery including the electrolyte, which has improved high-temperature properties.

To achieve the above object, according to an aspect of the present invention, there is provided an electrolyte for a lithium secondary battery including: an additive; an organic solvent; and a lithium salt, wherein the additive includes a compound represented by Formula 1 below or a compound represented by Formula 2 below.

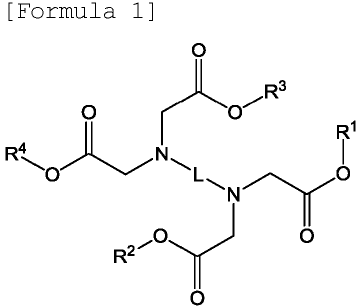

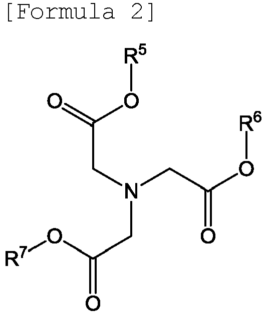

In Formula 1 and Formula 2 above, R¹ to R⁷ may be each independently a substituted or unsubstituted alkyl group having 1 to 10 carbon atoms, and L may be a substituted or unsubstituted alkylene group having 1 to 6 carbon atoms.

In some embodiments, the additive may include a compound represented by Formula 1-1 below.

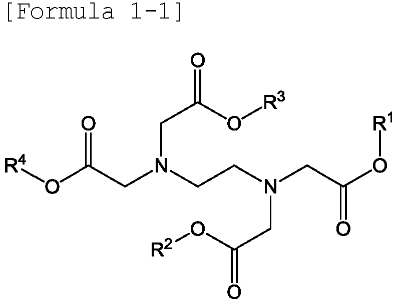

In Formula 1-1 above, R¹ to R⁴ may be each independently a substituted or unsubstituted alkyl group having 1 to 10 carbon atoms.

In some embodiments, R¹ to R⁷ in Formulas 1 and 2 above may be each independently an unsubstituted alkyl group having 1 to 3 carbon atoms.

In some embodiments, R¹ to R⁷ in Formulas 1 and 2 above may be a methyl group.

In some embodiments, a content of the additive may be 0.1 to 5% by weight based on a total weight of the electrolyte for a lithium secondary battery.

In some embodiments, the additive may include the compound represented by Formula 1 above and the compound represented by Formula 2 above together.

In some embodiments, a ratio of the content of the compound represented by Formula 2 above to the content of the compound represented by Formula 1 above may be 0.25 to 4 based on the weight.

In some embodiments, the organic solvent may include a linear carbonate solvent and a cyclic carbonate solvent.

In some embodiments, the electrolyte for a lithium secondary battery may further include at least one auxiliary additive selected from the group consisting of an unsaturated cyclic carbonate compound, a fluorine-substituted carbonate compound, a sultone compound, a cyclic sulfate compound and a phosphate compound.

In some embodiments, a content of the auxiliary additive may be 0.05 to 10% by weight based on the total weight of the electrolyte for a lithium secondary battery.

In some embodiments, a ratio of the content of the auxiliary additive to the content of the additive may be 0.1 to 10 based on the weight.

According to another aspect of the present invention, there is provided a lithium secondary battery including: a cathode; an anode disposed to face the cathode; and the electrolyte for a lithium secondary battery according to the above embodiments, in which the cathode and the anode are impregnated.

The electrolyte for a lithium secondary battery according to exemplary embodiments may include an additive represented by a specific formula to form a stable solid electrolyte interphase (SEI) on the surface of an electrode. Therefore, a decrease in the capacity and an increase in the resistance at a high temperature may be suppressed, and high-temperature lifespan properties of the lithium secondary battery may be improved.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a plan view schematically illustrating a lithium secondary battery according to exemplary embodiments;
FIG. 2 is a cross-sectional view schematically illustrating a lithium secondary battery according to exemplary embodiments;
FIG. 3 is a graph illustrating H-NMR results of a compound represented by Formula 1-2;
FIG. 4 is a graph illustrating H-NMR results of a compound represented by Formula 2-1; and
FIG. 5 is a graph illustrating capacity retention in lithium secondary batteries of Example 1, Example 3 and Comparative Example 1.

### [DETAILED DESCRIPTION OF THE INVENTION]

According to embodiments of the present disclosure, there are provided an electrolyte for a lithium secondary battery and a lithium secondary battery including the electrolyte for a lithium secondary battery.

The lithium secondary battery according to embodiments of the present disclosure may be widely applied to green technology fields such as an electric vehicle, and a battery charging station, as well as other solar power generation and wind power generation using the batteries. In addition, the lithium secondary battery according to embodiments of the present disclosure may be used in an eco-friendly electric vehicle, and a hybrid vehicle, etc., which are intended to prevent climate change by suppressing air pollution and greenhouse gas emissions.

If there is an isomer of a compound represented by a formula used in the present disclosure, the compound represented by the corresponding formula refers to the representative formula including the isomer.

The term "X compound" used in the present disclosure may refer to a compound including an X unit attached to a matrix, etc. of the "X compound" and a derivative thereof.

### <Electrolyte for a lithium secondary battery>

An electrolyte for a lithium secondary battery according to exemplary embodiments may include a lithium salt, an organic solvent and an additive.

Hereinafter, the electrolyte for a lithium secondary battery will be described in more detail.

### Additive

The additive may include a compound represented by Formula 1 below or a compound represented by Formula 2 below.

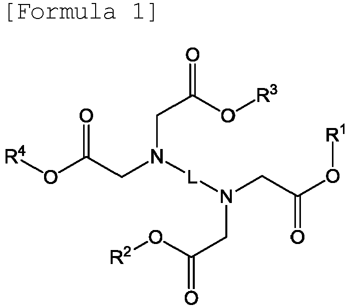

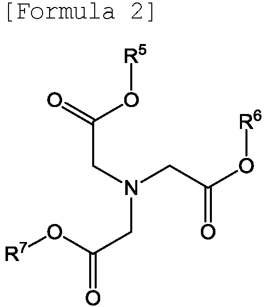

In Formulas 1 and 2 above, R¹ to R⁷ may be each independently a substituted or unsubstituted alkyl group having 1 to 10 carbon atoms.

For example, the alkyl group may mean a form in which one hydrogen atom is separated from alkane (CₙH₂ₙ₊₂). The portion where the hydrogen atom is separated may become a bonding site with an atom adjacent thereto.

As used herein, the term "substituted" may mean that at least one of hydrogen atoms of the alkyl group or alkylene group is substituted with a substituent, such that the substituent is further bonded to a carbon atom of the alkyl group or alkylene group.

For example, the substituent may be a halogen atom, an amino group, a nitro group, a cyano group, a sulfinyl group, a sulfonyl group, a carbonyl group, an alkyl group having 1 to 6 carbon atoms, an alkenyl group having 2 to 6 carbon atoms, an alkoxy group having 1 to 6 carbon atoms, a cycloalkyl group or heterocycloalkyl group having 3 to 7 carbon atoms.

In Formula 1 above, L may be a substituted or unsubstituted alkylene group having 1 to 6 carbon atoms.

For example, the alkylene group may mean a form in which two hydrogen atoms are separated from alkane (CₙH₂ₙ₊₂). The portion where the hydrogen atom is separated may become a bonding site with an atom adjacent thereto.

The additive includes a compound having a tertiary amine structure, and as ester groups are bonded around a nitrogen atom, a chelate for lithium ions may be formed more easily. Therefore, a lithium ion conductivity of the electrolyte may be improved, and output properties and rapid charging properties of the lithium secondary battery may be enhanced.

For example, a film having a low resistance may be formed on the surface of an electrode or active material from the amine compound. Accordingly, an increase in the initial resistance of the secondary battery may be suppressed, and rapid charging performance and lifespan properties may be significantly improved.

Therefore, when the electrolyte includes the compound represented by Formula 1 or Formula 2 as an additive, decomposition of the electrolyte may be prevented, and gas generation and an increase in the battery thickness may be significantly reduced.

In addition, as an alkyl group (R¹ to R⁷) is bonded to an end of the carboxylate bonded to the nitrogen atom, the high-temperature performance of the lithium secondary battery may be improved. For example, a thermally stable solid electrolyte interphase (SEI) may be formed on the surface of the electrode or active material from the amine compound. Therefore, even in the repeated charging and discharging, overvoltage, and high temperature environments, decomposition of the SEI film may be suppressed, and irreversible loss of the electrolyte may be prevented, thereby maintaining capacity and ionic conductivity of the lithium secondary battery high.

In some embodiments, L in Formula 1 above may be an unsubstituted alkylene group having 1 to 3 carbon atoms. For example, L may be a methylene group, an ethylene group or propylene group.

Preferably, L in Formula 1 is an ethylene group. For example, the additive may include a compound represented by Formula 1-1 below.

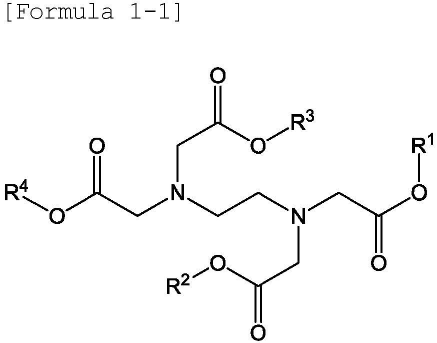

The number of carbon atoms between nitrogen atoms is appropriately adjusted, such that lithium ion conductivity and output properties may be further enhanced, while electrochemical stability, heat resistance, and flame retardancy of the electrolyte or a film formed from the electrolyte may be improved.

In one embodiment, in Formulas 1 and 2 above, R¹ to R⁷ may be each independently an unsubstituted alkyl group having 1 to 10 carbon atoms. As an unsubstituted hydrocarbon group is located at an end of the amine compound, high-temperature stability may be further improved. In addition, the number of carbon atoms in the alkyl group is adjusted in an appropriate range, such that the electrolyte and the film formed from the electrolyte may have a low electrical resistance and a high ionic conductivity.

In some embodiments, R¹ to R⁷ in Formulas 1 and 2 above may be each independently an unsubstituted alkyl group having 1 to 3 carbon atoms, or a methyl group.

For example, the additive may include a compound represented by Formula 1-2 below or Formula 2-1 below.

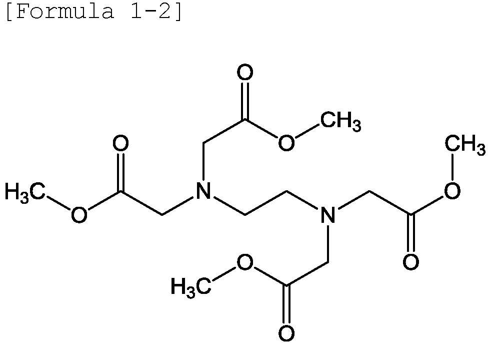

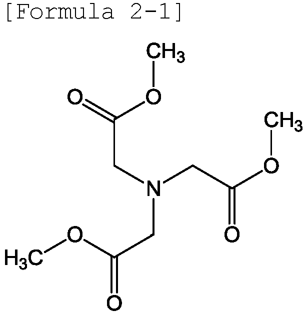

For example, as an alkyl group having a small number of carbon atoms is located at an end of the additive, steric hindrance and a decrease in the ionic conductivity may be prevented. Therefore, the flame retardancy and heat resistance of the electrolyte may be further improved by the additive, and the electrolyte may have a high lithium ion conductivity.

Therefore, it is possible to improve room temperature properties, rapid charging properties, and initial resistance properties along with high-temperature storage properties. In addition, gas generation may be reduced by suppressing decomposition of the electrolyte due to a reaction between the electrolyte and the electrode.

In one embodiment, a content of the additive may be 0.1% by weight ('wt%') or more, 0.2 wt% or more, 0.3 wt% or more, 0.4 wt% or more, 0.5 wt% or more, or 1 wt% or more based on a total weight of the electrolyte. Within the above range, a stable film may be uniformly formed on the electrode.

In one embodiment, the content of the additive may be 10 wt% or less, 9 wt% or less, 7 wt% or less, 6 wt% or less, 5 wt% or less, 4.5 wt% or less, 4 wt% or less, 3.5 wt% or less, 3 wt% or less, or 2 wt% or less based on the total weight of the electrolyte. Within the above range, the lithium ion conductivity in the electrolyte and activity of an electrode active material may be improved.

For example, the content of the additive may be 0.1 to 5 wt%, or 0.2 to 2 wt% based on the total weight of the electrolyte. Within the above range, movement of lithium ions and activity of a cathode active material are not excessively inhibited, and capacity retention may be improved even in the high temperature environment, as well as electrode thickness and resistance may be maintained low.

In some embodiments, the additive may include the compound represented by Formula 1 above and the compound represented by Formula 2 above together. As the affinity for lithium ions may be increased to improve the ionic conductivity, and electrochemical and thermal stabilities of the film may be further improved.

In one embodiment, a ratio of the content of the compound represented by Formula 2 above to the content of the compound represented by Formula 1 above may be 0.25 to 4, 0.3 to 4, 0.5 to 2, or 0.5 to 1.5 based on the weight. Within the above range, the heat resistance and flame retardancy of the lithium secondary battery may be improved while the output properties thereof may be further improved.

### Auxiliary additive

The electrolyte for a lithium secondary battery according to exemplary embodiments may further include an auxiliary additive.

When adding the auxiliary additive, durability and stability of the electrode may be further improved. The auxiliary additive may be included in an appropriate amount within a range that does not inhibit the movement of lithium ions in the electrolyte.

In one embodiment, the auxiliary additive may include, for example, an unsaturated cyclic carbonate compound, a fluorine-substituted carbonate compound, a sultone compound, a cyclic sulfate compound and a phosphate compound.

In one embodiment, a content of the auxiliary additive may be, for example, 10 wt% or less, 9 wt% or less, 8 wt% or less, 7 wt% or less, 6 wt% or less, 5 wt% or less, 4 wt% or less, 3 wt% or less, 2 wt% or less, or 1 wt% or less based on the total weight of the electrolyte for a lithium secondary battery in consideration of action with the compound represented by Formula 1 or Formula 2 above.

In one embodiment, the content of the auxiliary additive may be 0.01 wt% or more, 0.02 wt% or more, 0.03 wt% or more, 0.05 wt% or more, 0.1 wt% or more, 0.2 wt% or more, 0.3 wt% or more, 0.4 wt% or more, or 0.5 wt% or more based on the total weight of the electrolyte for a lithium secondary battery. Within the above range, a more stable film may be formed on the surface of the electrode.

Preferably, the content of the auxiliary additive is about 0.05 to 10 wt%, and more preferably 0.1 to 5 wt% based on the total weight of the electrolyte. Within the above range, the durability of an electrode protective film may be improved without inhibiting the action of the main additive, and the high-temperature storage and lifespan properties may be further improved.

In one embodiment, a ratio of the weight of the auxiliary additive to the weight of the additive in the electrolyte may be 0.1 to 10, greater than 1 and 10 or less, or 5 to 10 or less. The content of the auxiliary additive to the main additive is adjusted in an appropriate range, such that cycle properties and high-temperature storage properties of the lithium secondary battery may be improved due to an interaction between the additives.

The unsaturated cyclic carbonate compound may include vinylene carbonate (VC), vinyl ethylene carbonate (VEC) and the like.

The fluorine-substituted carbonate compound may include fluoroethylene carbonate (FEC).

The sultone compound may include 1,3-propane sultone, 1,3-propene sultone, 1,4-butane sultone and the like.

The cyclic sulfate compound may include 1,2-ethylene sulfate, 1,2-propylene sulfate and the like.

The phosphate compound may include lithium bis(oxalato)phosphate as an oxalatophosphate compound.

In some embodiments, as the auxiliary additive, the fluorine-substituted carbonate compound, the sultone compound, the cyclic sulfate compound and the oxalate compound may be used together.

### Organic solvent and lithium salt

The organic solvent may include, for example, an organic compound which has sufficient solubility to the lithium salt, the additive, and/or the auxiliary additive, and does not have reactivity in the battery.

For example, the organic solvent may include at least one of a carbonate solvent, an ester solvent, an ether solvent, a ketone solvent, an alcohol solvent and an aprotic solvent.

In one embodiment, the organic solvent may include a carbonate solvent.

In some embodiments, the carbonate solvent may include a linear carbonate solvent and a cyclic carbonate solvent.

For example, the linear carbonate solvent may include at least one of dimethyl carbonate (DMC), ethyl methyl carbonate (EMC), diethyl carbonate (DEC), methyl propyl carbonate, ethyl propyl carbonate and dipropyl carbonate.

For example, the cyclic carbonate solvent may include at least one of ethylene carbonate (EC), propylene carbonate (PC) and butylene carbonate.

In some embodiments, the content of the linear carbonate solvent may be greater than the content of the cyclic carbonate solvent based on the volume of the organic solvent.

For example, a mixing volume ratio of the linear carbonate solvent and the cyclic carbonate solvent may be 1:1 to 9:1, and preferably 1.5:1 to 4:1.

For example, the ester solvent may include at least one of methyl acetate (MA), ethyl acetate (EA), n-propyl acetate (n-PA), 1,1-dimethylethyl acetate (DMEA), methyl propionate (MP) and ethyl propionate (EP).

For example, the ether solvent may include at least one of dibutyl ether, tetraethylene glycol dimethyl ether (TEGDME), diethylene glycol dimethyl ether (DEGDME), dimethoxyethane, tetrahydrofuran (THF) and 2-methyltetrahydrofuran.

For example, the ketone solvent may include cyclohexanone.

For example, the alcohol solvent may include at least one of ethyl alcohol and isopropyl alcohol.

For example, the aprotic solvent may include at least one of a nitrile solvent, an amide solvent (e.g., dimethylformamide), a dioxolane solvent (e.g., 1,3-dioxolane), and a sulfolane solvent

The electrolyte includes a lithium salt, and the lithium salt may be expressed as Li⁺X⁻.

For example, the anion (X-) of the lithium salt may be at least one of F⁻, Cl⁻, Br⁻, I⁻, NO₃⁻, N(CN)₂⁻, BF₄⁻, ClO₄⁻, PF₆⁻, (CF₃)₂PF₄⁻, (CF₃)₃PF₃⁻, (CF₃)₄PF₂⁻, (CF₃)₅PF⁻, (CF₃)₆P⁻, CF₃SO₃⁻, CF₃CF₂SO₃⁻, (CF₃SO₂)₂N⁻, (FSO₂)₂N⁻, CF₃CF₂(CF₃)₂CO⁻, (CF₃SO₂)₂CH⁻, (SF₅)₃C⁻ , (CF₃SO₂)₃C⁻, CF₃(CF₂)₇SO₃⁻, CF₃CO₂⁻, CH₃CO₂⁻, SCN⁻ and (CF₃CF₂SO₂)₂N⁻.

In some embodiments, the lithium salt may include at least one of LiBF₄ and LiPF₆.

In one embodiment, a concentration of the lithium salt may be 0.01 to 5 M, preferably 0.01 to 2 M relative to the organic solvent. Within the above concentration range, lithium ions and electrons may move smoothly within the electrolyte, and the rapid charging properties and output properties of the lithium secondary battery may be further improved.

### <Lithium secondary battery>

The lithium secondary battery according to exemplary embodiments may include a cathode, an anode, a separation membrane interposed between the cathode and the anode, and the above-described electrolyte for a lithium secondary battery.

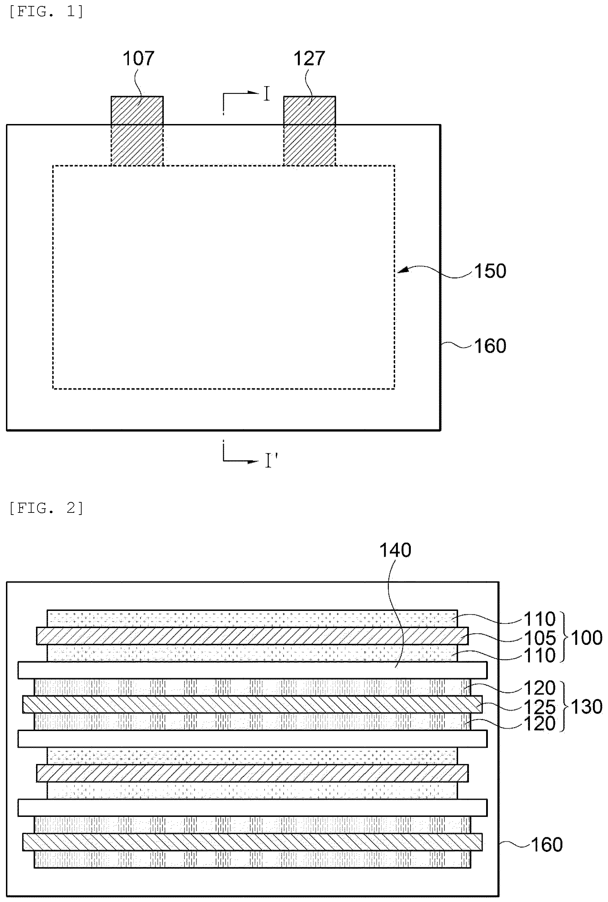

FIGS. 1 and 2 are a schematic plan view and a cross-sectional view, respectively, illustrating a lithium secondary battery according to exemplary embodiments. FIG. 2 is a cross-sectional view taken on line I-I' in FIG. 1.

Referring to FIGS. 1 and 2, the lithium secondary battery may include a cathode 100 and an anode 130 disposed to face the cathode 100.

For example, the cathode 100 may include a cathode current collector 105 and a cathode active material layer 110 on the cathode current collector 105.

For example, the cathode active material layer 110 may include a cathode active material, a cathode binder and a conductive material, as necessary.

For example, the cathode 100 may be prepared by mixing and stirring the cathode active material, the cathode binder, and the conductive material, etc. in a dispersion medium to prepare a cathode slurry, and then applying the cathode slurry to the cathode current collector 105, followed by drying and rolling the same.

For example, the cathode current collector 105 may include stainless steel, nickel, aluminum, titanium, copper, or an alloy thereof, and preferably, includes aluminum or an aluminum alloy.

For example, the cathode active material may include lithium metal oxide particles capable of reversibly intercalating and deintercalating lithium ions.

In one embodiment, the cathode active material may include lithium metal oxide particles containing nickel.

Examples of the cathode active material may include one or more compounds selected from lithium cobalt oxide, lithium manganese oxide, lithium nickel oxide or lithium composite oxide. For example, the cathode active material may include a layered compound such as lithium cobalt oxide (LiCoO₂) or lithium nickel oxide (LiNiO₂), or lithium manganese oxide such as LiMnO₃, LiMn₂O₃ and LiMnO₂; lithium copper oxide (Li₂CuO₂); lithium iron phosphate oxide and the like.

In one embodiment, the lithium metal oxide particles may be represented by Formula 3 below.

[Formula 3] LiₐNi_{b}M₁-_{b}O₂

In Formula 3, a and b may be in a range of 0.95≤a≤1.08, and b≥0.5, respectively, and M may be at least one element selected from Na, Mg, Ca, Y, ti, Zr, Hf, V, Nb, ta, Cr, Mo, W, Mn, Co, Fe, Cu, Ag, Zn, B, Al, Ga, C, Si, Sn, Ba and Zr.

In one embodiment, the cathode active material includes nickel (Ni), and may further include at least one of cobalt (Co) and manganese (Mn). For example, nickel-cobalt-manganese (NCM)-based lithium oxide may be used.

For example, nickel (Ni) may be provided as a metal associated with the capacity of the lithium secondary battery. The higher the content of nickel, the better the capacity and output of the lithium secondary battery, but if the content of nickel is excessively increased, the lifespan of the battery may be decreased, and it may be disadvantageous in terms of mechanical and electrical stabilities.

In one embodiment, the conductivity or resistance of the lithium secondary battery may be improved by cobalt (Co), as well as the mechanical and electrical stabilities of the lithium secondary battery may be improved by manganese (Mn).

The chemical structure represented by Formula 3 shows a bonding relationship between elements included in the lattice structure or crystal structure of the cathode active material, and does not exclude other additional elements. For example, M may be provided as a main active element of the cathode active material. Here, it should be understood that Formula 3 is provided to express the bonding relationship between the main active elements, and is a formula encompassing introduction and substitution of the additional elements.

In one embodiment, the inventive material may further include auxiliary elements which are added to the main active elements, thus to enhance chemical stability of the cathode active material or the crystal structure. The auxiliary element may be incorporated into the crystal structure together to form a bond, and it should be understood that this case is also included within the chemical structure represented by Formula 3.

For example, the cathode binder may include an organic binder such as polyvinylidene fluoride (PVDF), vinylidene fluoride-hexafluoropropylene copolymer (PVDF-co-HFP), polyacrylonitrile, polymethyl methacrylate, etc.; or an aqueous binder such as styrene-butadiene rubber (SBR). In addition, for example, the cathode binder may be used together with a thickener such as carboxymethyl cellulose (CMC).

For example, the conductive material may include a carbon-based conductive material such as graphite, carbon black, graphene, and carbon nanotubes; or a metal-based conductive material including tin, tin oxide, titanium oxide, or a perovskite material such as LaSrCoO₃, and LaSrMnO₃.

For example, the anode 130 may include an anode current collector 125 and an anode active material layer 120 on the anode current collector 125.

For example, the anode active material layer 120 may include an anode active material, an anode binder, and a conductive material, as necessary.

For example, the anode 130 may be prepared by mixing and stirring the anode active material, the anode binder, the conductive material, etc. in a solvent to prepare an anode slurry, and then applying the anode slurry to the anode current collector 125, followed by drying and rolling the same.

For example, the anode current collector 125 may include gold, stainless steel, nickel, aluminum, titanium, copper or an alloy thereof, and preferably, includes copper or a copper alloy.

For example, the anode active material may be a material capable of intercalating and deintercalating lithium ions. For example, the anode active material may include a lithium alloy, a carbon-based material, a silicon-based material and the like.

For example, the lithium alloy may include aluminum, zinc, bismuth, cadmium, antimony, silicon, lead, tin, gallium, indium and the like.

For example, the carbon-based active material may include crystalline carbon, amorphous carbon, carbon composite, carbon fiber and the like.

For example, the amorphous carbon may include hard carbon, cokes, mesocarbon microbead (MCMB) calcined at 1500°C or lower, mesophase pitch-based carbon fiber (MPCF) or the like. For example, the crystalline carbon may include natural graphite, graphite cokes, graphite MCMB, graphite MPCF and the like.

For example, the silicon-based active material may include Si, SiOₓ (0<x<2), Si/C, SiO/C, Si-metal and the like. In this case, it is possible to implement a lithium secondary battery having a high capacity.

The silicon-based material may have a large change in the volume during reacting with lithium. Therefore, when the anode active material includes the silicon-based material, the thickness of the battery may be increased due to the repeated charging and discharging, and short circuit and ignition may occur. The lithium secondary battery according to exemplary embodiments may include the above-described electrolyte to relieve a thickness increase rate of the battery.

The anode binder and the conductive material may be substantially the same as or similar to the above-described cathode binder and the conductive material. For example, the anode binder may be an aqueous binder such as styrene-butadiene rubber (SBR). In addition, for example, the anode binder may be used together with a thickener such as carboxymethyl cellulose (CMC).

For example, a separation membrane 140 may be interposed between the cathode 100 and the anode 130.

In some embodiments, the anode 130 may have an area greater than that of the cathode 100. In this case, lithium ions generated from the cathode 100 may smoothly move to the anode 130 without precipitation in the middle.

For example, the separation membrane 140 may include a porous polymer film made of a polyolefin polymer such as ethylene homopolymer, propylene homopolymer, ethylene/butene copolymer, ethylene/hexene copolymer, ethylene/methacrylate copolymer or the like. Alternatively, for example, the separation membrane 140 may include a nonwoven fabric made of glass fiber having a high melting point, polyethylene terephthalate fiber or the like.

For example, an electrode cell may be formed including the cathode 100, the anode 130 and the separation membrane 140.

For example, a plurality of electrode cells may be stacked to form an electrode assembly 150. For example, the electrode assembly 150 may be formed by winding, laminating, folding, etc. the separation membrane 140.

The lithium secondary battery according to exemplary embodiments may include: a cathode lead 107 connected to the cathode 100 and protruding to an outside of a case 160; and an anode lead 127 connected to the anode 130 and protruding to the outside of the case 160.

For example, the cathode lead 107 may be electrically connected to the cathode current collector 105. The anode lead 127 may be electrically connected to the anode current collector 125.

For example, the cathode current collector 105 may include a protrusion part (cathode tab, not illustrated) on one side. The cathode active material layer 110 may not be formed on the cathode tab. The cathode tab may be formed integrally with the cathode current collector 105 or may be connected thereto by welding or the like. The cathode current collector 105 and the cathode lead 107 may be electrically connected with each other through the cathode tab.

Similarly, the anode current collector 125 may include a protrusion part (anode tab, not illustrated) on one side. The anode active material layer 120 may not be formed on the anode tab. The anode tab may be formed integrally with the anode current collector 125 or may be connected thereto by welding or the like. The anode current collector 125 and the anode lead 127 may be electrically connected with each other through the anode tab.

According to exemplary embodiments, the electrode assembly 150 and the above-described electrolyte may be housed together in the case 160 to define a lithium secondary battery. The lithium secondary battery may be manufactured, for example, in a cylindrical shape using a can, a square shape, a pouch shape or a coin shape, etc.

Hereinafter, specific experimental examples are proposed to facilitate understanding of the present invention.

### Examples and Comparative Examples

### (1) Synthesis Example 1: Tetramethyl 2,2',2",2‴-[ethane-1,2-diylbis (azanetriyl)]tetraacetate (additive A, Formula 1-2)

Ethylenediaminetetraacetic acid (2.9 g, 10 mmol) and methanol (100 mL) were sequentially input into a round-bottom flask, followed by cooling the same to 0°C. Thionyl chloride (5.8 mL, 80 mmol) was slowly added dropwise to the mixture while maintaining 0°C, and then stirred at room temperature for 16 hours. After the reaction was completed, the pressure of the mixture was reduced to remove the remaining thionyl chloride and methanol. The remaining mixture after reducing the pressure was dissolved again in ether (50 mL) as a solvent, and sodium bicarbonate saturated solution (50 mL) was slowly added dropwise and stirred for 1 hour to neutralize the mixture. After completion of the reaction, the organic layer was separated to remove moisture through calcium carbonate treatment. Thereafter, the solvent in the organic layer was removed by reducing the pressure and dried to obtain 1.46 g of a compound as a colorless liquid sample. 1H-NMR results of the obtained compound are as follows.
¹H-NMR (CDCl₃, 500 mHz): 3.69(s, 12H), 3.61(s, 8H), 2.89(s, 4H)

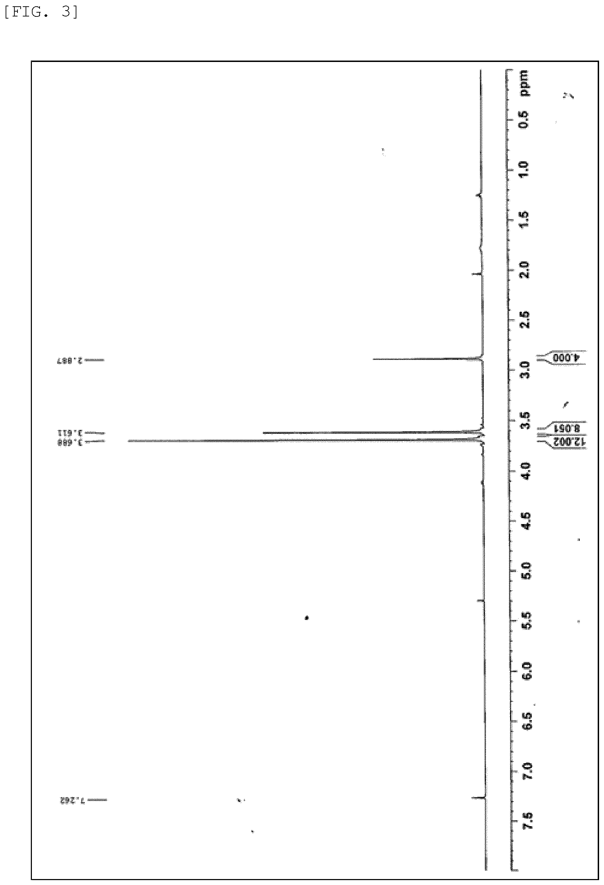

FIG. 3 is a graph illustrating the H-NMR results of the compound represented by Formula 1-2.

### (2) Synthesis Example 2: Trimethyl 2,2',2"-nitrilotriacetate (additive B, Formula 2-1)

Nitrilotriacetic acid (1.9 g, 10 mmol) and methanol (100 mL) were sequentially input into a round-bottom flask, followed by cooling the same to 0°C. Thionyl chloride (4.4 mL, 60 mmol) was slowly added dropwise to the mixture while maintaining 0°C, and then stirred at room temperature for 16 hours. After completion of the reaction, the pressure of the mixture was reduced to remove the remaining thionyl chloride and methanol. The remaining mixture after reducing the pressure was dissolved again in ether (50 mL) as a solvent, and sodium bicarbonate saturated solution (50 mL) was slowly added dropwise and stirred for 1 hour to neutralize the mixture. After completion of the reaction, the organic layer was separated to remove moisture through calcium carbonate treatment. Thereafter, the solvent in the organic layer was removed by reducing the pressure and dried to obtain 1.65 g of a compound as a transparent yellowish liquid sample. 1H-NMR results of the obtained compound are as follows.
¹H-NMR (CDCl₃, 500 mHz): 3.70(s, 9H), 3.67(s, 6H)

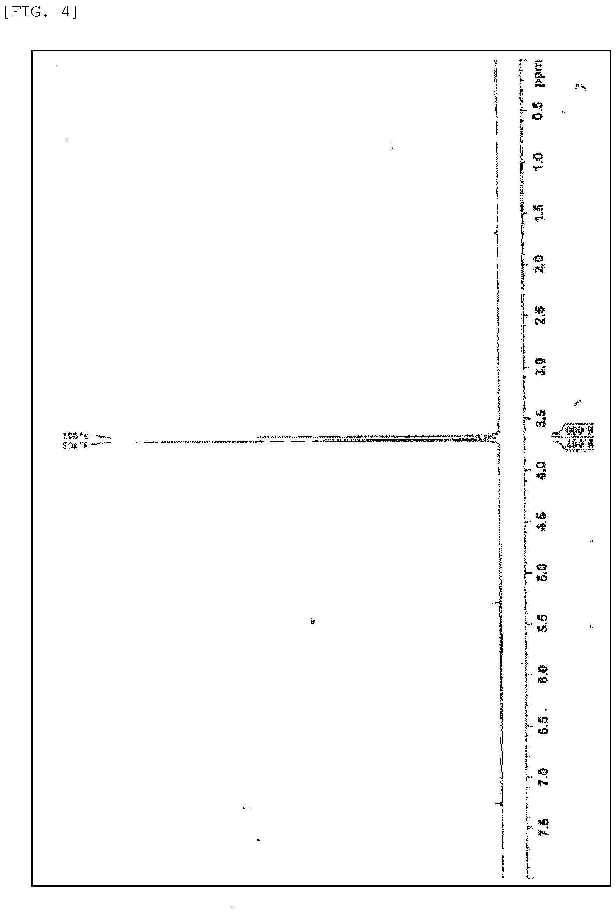

FIG. 4 is a graph illustrating the H-NMR results of the compound represented by Formula 2-1.

### (3) Preparation of electrolyte

1 M LiPF₆ solution (a mixed solvent of EC/EMC in a volume ratio of 25:75) was prepared.

Electrolytes of the examples and comparative examples were prepared by adding additives and auxiliary additives in the contents (wt%) described in Table 1 below to the LiPF₆ solution based on a total weight of the electrolyte, and mixing with each other. In the case of Examples 5 to 10, the additive A and the additive B were mixed together.

### (4) Manufacturing of lithium secondary batteries

A cathode slurry was prepared by mixing and dispersing a cathode active material of Li[Ni_{0.8}Co_{0.1}Mn_{0.1}]O₂, carbon black, and polyvinylidene fluoride (PVDF) in N-methyl pyrrolidone(NMP) in a weight ratio of 98:1:1.

The cathode slurry was uniformly applied to an aluminum foil having a thickness of 12 µm, followed by drying and rolling the same to prepare a cathode.

An anode slurry was prepared by mixing and dispersing an anode active material in which artificial graphite and natural graphite are mixed in a weight ratio of 7:3, a conductive material, styrene-butadiene rubber (SBR) and carboxymethyl cellulose (CMC) in water in a weight ratio of 96:3:1:1. The anode slurry was uniformly applied to a copper foil having a thickness of 8 µm, followed by drying and rolling the same to prepare an anode.

An electrode assembly was manufactured by repeatedly stacking the manufactured cathodes and anodes alternately, with a separator (thickness 13 µm, polyethylene film) interposed between the cathode and anode.

The electrode assembly was placed in a pouch, the electrolyte prepared in (3) above was injected, sealed, and impregnated for 12 hours to manufacture a lithium secondary battery.

**[TABLE 1]**

| | Additive (content) | Auxiliary additive (wt%) | | |
|---|---|---|---|---|
| | | FEC | PS | PRS |
| Example 1 | Additive A (1.0 wt%) | 1 | 0.5 | 0.5 |
| Example 2 | Additive A (0.5 wt%) | 1 | 0.5 | 0.5 |
| Example 3 | Additive B (1.0 wt%) | 1 | 0.5 | 0.5 |
| Example 4 | Additive B (0.5 wt%) | 1 | 0.5 | 0.5 |
| Example 5 | Additive A (0.1 wt%) Additive B (0.9 wt%) | 1 | 0.5 | 0.5 |
| Example 6 | Additive A (0.2 wt%) Additive B (0.8 wt%) | 1 | 0.5 | 0.5 |
| Example 7 | Additive A (0.4 wt%) Additive B (0.6 wt%) | 1 | 0.5 | 0.5 |
| Example 8 | Additive A (0.6 wt%) Additive B (0.4 wt%) | 1 | 0.5 | 0.5 |
| Example 9 | Additive A (0.8 wt%) Additive B (0.2 wt%) | 1 | 0.5 | 0.5 |
| Example 10 | Additive A (0.9 wt%) Additive B (0.1 wt%) | 1 | 0.5 | 0.5 |
| Comparative Example 1 | - | 1 | 0.5 | 0.5 |
| Comparative Example 2 | Additive C, 1.0 wt% | 1 | 0.5 | 0.5 |
| Comparative Example 3 | Additive D, 1.0 wt% | 1 | 0.5 | 0.5 |

The components described in Table 1 are as follows.
Additive A: The compound represented by formula 1-2 above
Additive B: The compound represented by Formula 2-1 above
Additive C: A compound represented by Formula 4 below: 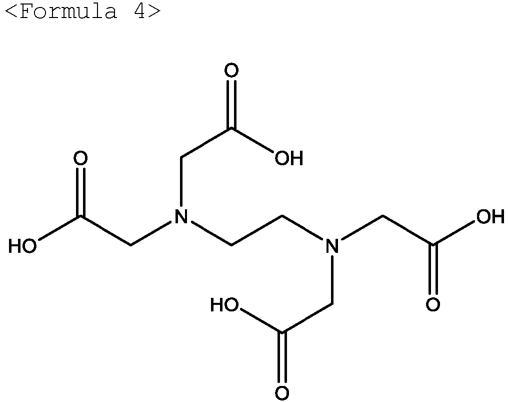
Additive D: A compound represented by Formula 5 below: 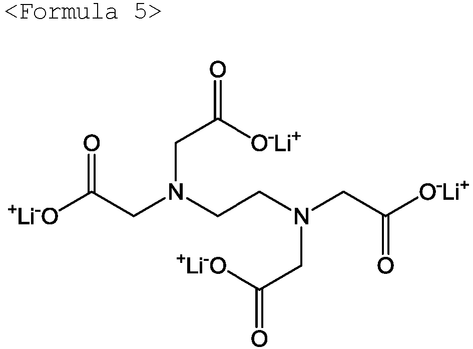
FEC: Fluoroethylene carbonate
PS: 1,3-Propane sultone
PRS: Prop-1-ene-1,3-sultone

### <Experimental example>

### (1) Evaluation of solubility

After the electrolytes according to the examples and comparative examples were left at room temperature for 3 hours, it was observed visually whether precipitation or layer separation occurs. If the precipitation or layer separation did not occur, evaluation results were marked as "O," whereas if it did occur, the evaluation results were marked as "X" in a Table 2.

### (2) Measurement of initial resistance (DCIR)

The lithium secondary batteries of the examples and comparative examples were subjected to 0.5C CC/CV charge (4.2 V 0.05C CUT-OFF) to state of charge (SOC) 60% at room temperature (25°C), and then 0.5C CC discharge. Thereafter, the lithium secondary batteries were discharged and supplementary charged for 10 seconds, while changing C-rate to 0.2C 0.5C, 1C, 1.5C, 2C and 2.5C at the SOC 60% point, respectively, and the initial resistance was measured.

### (3) Evaluation of high-temperature lifespan at 45°C

### (3-1) Capacity retention at 45°C

900 cycles of charging/discharging were repeatedly performed on the lithium secondary batteries of the examples and comparative examples in a way that executing charging at 45°C to SOC 96% at a C-rate of 1.0C and discharging to SOC 2% at a C-rate of 1.0C were set to be one cycle. In this case, the initial discharge capacity at the first cycle was measured, and the capacity retention of the high-temperature lifespan was determined by dividing the discharge capacity at 900 cycles by the discharge capacity at the first cycle.

### (3-2) Resistance increase rate at 45°C

The lithium secondary batteries of the examples and comparative examples were charged to SOC 60% every time when they reached 100 cycles from the first cycle at 45°C, and then discharged and supplementary charged for 10 seconds, while changing C-rate to 0.2C 0.5C, 1C, 1.5C, 2C and 2.5C at the SOC 60% point, respectively, followed by measuring the internal resistance. Thereafter, the resistance increase rate of the high-temperature lifespan was determined by dividing the resistance value at 900 cycles by the resistance value at the first cycle.

Evaluation results are shown in Table 2 below.

**[TABLE 2]**

| | Evaluation of solubility | Initial performance at room temperature | Lifespan properties at 45°C and 900 cycles | | |
|---|---|---|---|---|---|
| | | Initial DCIR (mΩ) | Resistance increase rate (%) | Capacity (mAh) | Capacity retention (%) |
| Example 1 | ○ | 37.1 | 104 | 1239 | 76.0 |
| Example 2 | ○ | 39.2 | 101 | 1211 | 72.5 |
| Example 3 | ○ | 41.0 | 107 | 1323 | 81.8 |
| Example 4 | ○ | 39.8 | 106 | 1289 | 79.6 |
| Example 5 | ○ | 41.1 | 110 | 1311 | 80.1 |
| Example 6 | ○ | 40.2 | 109 | 1299 | 79.8 |
| Example 7 | ○ | 36.5 | 102 | 1320 | 81.5 |
| Example 8 | ○ | 35.8 | 107 | 1271 | 78.9 |
| Example 9 | ○ | 38.9 | 103 | 1256 | 77.7 |
| Example 10 | ○ | 36.1 | 103 | 1301 | 79.9 |
| Comparative Example 1 | ○ | 39.5 | 111 | 1170 | 70.1 |
| Comparative Example 2 | X | Evaluation impossible | | | |
| Comparative Example 3 | X | Evaluation impossible | | | |

Referring to Table 2, in the case of examples including the additive A or additive B, the initial resistance at room temperature and the resistance increase rate at high temperature were low, or the high-temperature capacity retention was improved. In the case of examples including the additive A and additive B together, the initial resistance and high-temperature resistance increase rate, or high-temperature capacity and high-temperature capacity retention were improved together.

In the case of Comparative Example 1 including only auxiliary additives, the high-temperature capacity and high temperature life performance (at 45°C) were reduced.

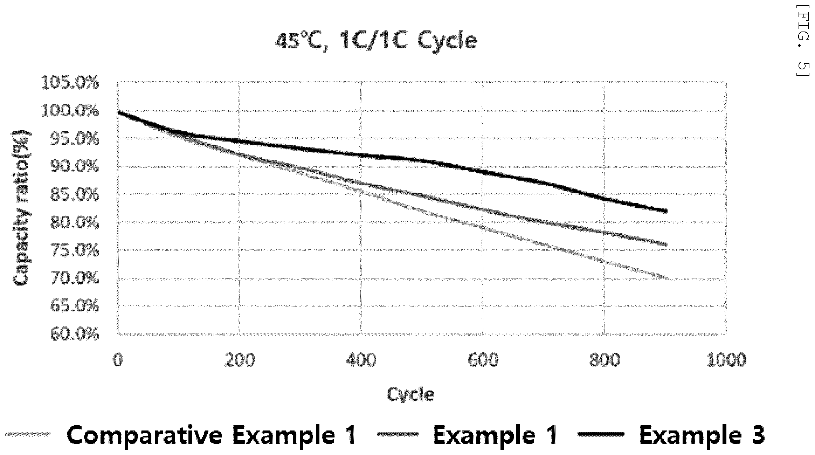

FIG. 5 is a graph illustrating the high-temperature capacity retention according to the cycle. Referring to FIG. 5, the capacity in the lithium secondary battery of Comparative Example 1 was decreased rapidly as the number of cycles was increased compared to the lithium secondary batteries of Examples 1 and 3.

The electrolytes of Comparative Examples 2 and 3 include an amine compound (additive C) having a carboxyl group at an end thereof and an amine compound (additive D) containing a lithium salt as additives, respectively. The solubility of the additive C and the additive D in the electrolyte was very low, such that the composition after mixing was non-uniform, and performance evaluation for the lithium secondary battery was impossible.

## Claims

1. An electrolyte for a lithium secondary battery comprising:
an additive which comprises a compound represented by Formula 1 below or a compound represented by Formula 2 below;
an organic solvent; and
a lithium salt: 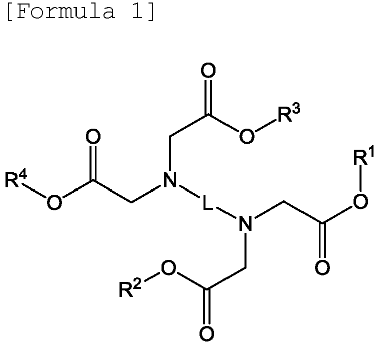 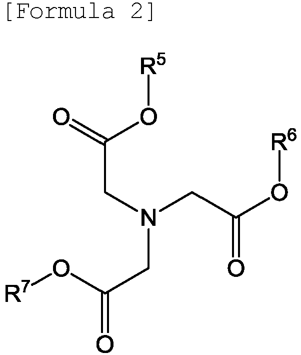
(in Formula 1 and Formula 2 above,
R¹ to R⁷ are each independently a substituted or unsubstituted alkyl group having 1 to 10 carbon atoms, and L is a substituted or unsubstituted alkylene group having 1 to 6 carbon atoms).

2. The electrolyte for a lithium secondary battery according to claim 1, wherein the additive comprises a compound represented by Formula 1-1 below: 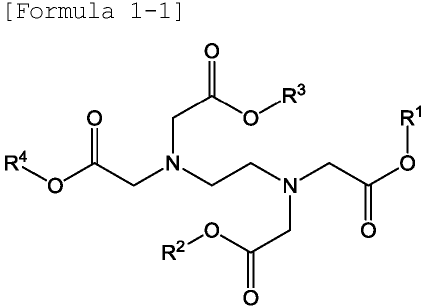 (in Formula 1-1 above, R¹ to R⁴ are each independently a substituted or unsubstituted alkyl group having 1 to 10 carbon atoms) .

3. The electrolyte for a lithium secondary battery according to claims 1 or 2, wherein R¹ to R⁷ in Formulas 1 and 2 above are each independently an unsubstituted alkyl group having 1 to 3 carbon atoms.

4. The electrolyte for a lithium secondary battery according to one of claims 1 to 3, wherein R¹ to R⁷ in Formulas 1 and 2 above are a methyl group.

5. The electrolyte for a lithium secondary battery according to one of claims 1 to 4, wherein the additive comprises a compound represented by Formula 1-2 below or a compound represented Formula 2-1 below:

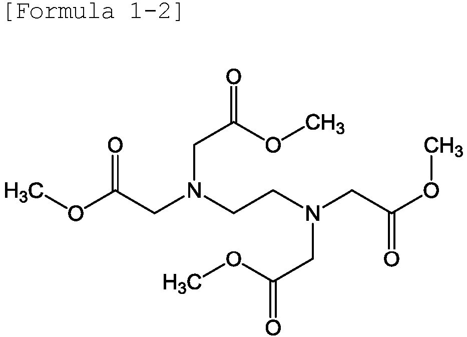

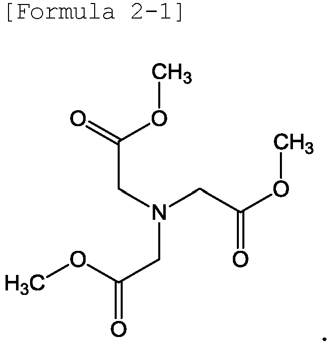

6. The electrolyte for a lithium secondary battery according to one of claims 1 to 5, wherein a content of the additive is 0.1 to 5% by weight based on a total weight of the electrolyte for a lithium secondary battery.

7. The electrolyte for a lithium secondary battery according to one of claims 1 to 6, wherein the additive comprises the compound represented by Formula 1 above and the compound represented by Formula 2 above together.

8. The electrolyte for a lithium secondary battery according to claim 7, wherein a ratio of the content of the compound represented by Formula 2 above to the content of the compound represented by Formula 1 above is 0.25 to 4 based on the weight.

9. The electrolyte for a lithium secondary battery according to one of claims 1 to 8, wherein the organic solvent comprises a linear carbonate solvent and a cyclic carbonate solvent.

10. The electrolyte for a lithium secondary battery according to one of claims 1 to 9, further comprising at least one auxiliary additive selected from the group consisting of an unsaturated cyclic carbonate compound, a fluorine-substituted carbonate compound, a sultone compound, a cyclic sulfate compound and a phosphate compound.

11. The electrolyte for a lithium secondary battery according to claim 10, wherein a content of the auxiliary additive is 0.05 to 10% by weight based on the total weight of the electrolyte for a lithium secondary battery.

12. The electrolyte for a lithium secondary battery according to claims 10 or 11, wherein a ratio of the content of the auxiliary additive to the content of the additive is 0.1 to 10 based on the weight.

13. A lithium secondary battery comprising:
a cathode;
an anode disposed to face the cathode; and
the electrolyte for a lithium secondary battery according to one of claims 1 to 12, in which the cathode and the anode are impregnated.

## Patentansprüche

1. Elektrolyt für eine Lithium-Sekundärbatterie, umfassend:
ein Additiv, das eine durch die nachstehende Formel 1 dargestellte Verbindung oder eine durch die nachstehende Formel 2 dargestellte Verbindung umfasst;
ein organisches Lösungsmittel; und
ein Lithiumsalz: 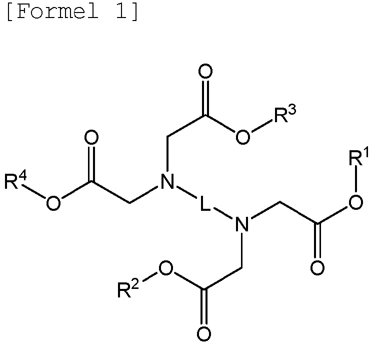 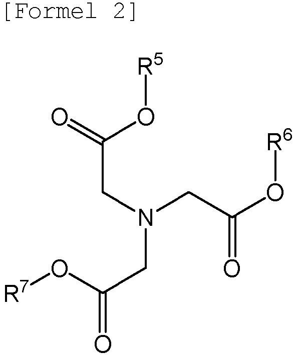
(in Formel 1 und Formel 2 oben gilt,
R¹ bis R⁷ sind jeweils unabhängig voneinander eine substituierte oder unsubstituierte Alkylgruppe mit 1 bis 10 Kohlenstoffatomen, und L ist eine substituierte oder unsubstituierte Alkylengruppe mit 1 bis 6 Kohlenstoffatomen).

2. Elektrolyt für eine Lithium-Sekundärbatterie nach Anspruch 1, wobei das Additiv eine durch die nachstehende Formel 1-1 dargestellte Verbindung umfasst: 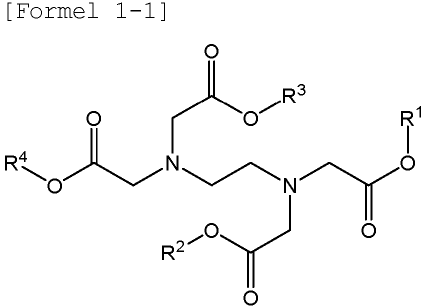 (in der obigen Formel 1-1 sind R¹ bis R⁴ jeweils unabhängig voneinander eine substituierte oder unsubstituierte Alkylgruppe mit 1 bis 10 Kohlenstoffatomen).

3. Elektrolyt für eine Lithium-Sekundärbatterie nach Anspruch 1 oder 2, wobei R¹ bis R⁷ in den obigen Formeln 1 und 2 jeweils unabhängig voneinander eine unsubstituierte Alkylgruppe mit 1 bis 3 Kohlenstoffatomen sind.

4. Elektrolyt für eine Lithium-Sekundärbatterie nach einem der Ansprüche 1 bis 3, wobei R¹ bis R⁷ in den obigen Formeln 1 und 2 eine Methylgruppe sind.

5. Elektrolyt für eine Lithium-Sekundärbatterie nach einem der Ansprüche 1 bis 4, wobei das Additiv eine Verbindung der nachstehenden Formel 1-2 oder eine Verbindung der nachstehenden Formel 2-1 umfasst:

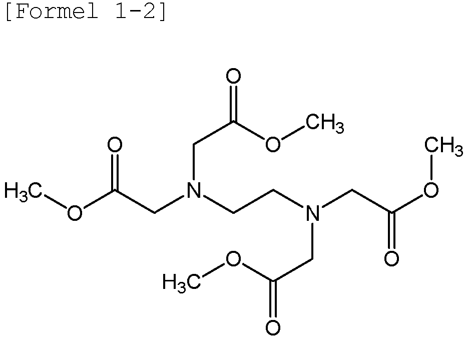

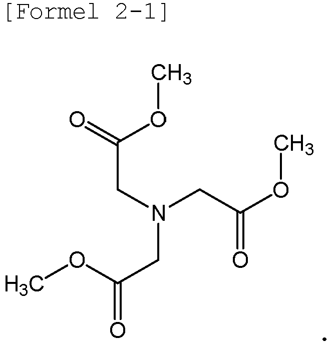

6. Elektrolyt für eine Lithium-Sekundärbatterie nach einem der Ansprüche 1 bis 5, wobei der Gehalt an dem Additiv 0,1 bis 5 Gew.-%, bezogen auf das Gesamtgewicht des Elektrolyten für eine Lithium-Sekundärbatterie, beträgt.

7. Elektrolyt für eine Lithium-Sekundärbatterie nach einem der Ansprüche 1 bis 6, wobei das Additiv die durch die obige Formel 1 dargestellte Verbindung und die durch die obige Formel 2 dargestellte Verbindung zusammen umfasst.

8. Elektrolyt für eine Lithium-Sekundärbatterie nach Anspruch 7, wobei das Verhältnis des Gehalts der durch die obige Formel 2 dargestellten Verbindung zu dem Gehalt der durch die obige Formel 1 dargestellten Verbindung 0,25 bis 4, bezogen auf das Gewicht, beträgt.

9. Elektrolyt für eine Lithium-Sekundärbatterie nach einem der Ansprüche 1 bis 8, wobei das organische Lösungsmittel ein lineares Carbonat-Lösungsmittel und ein zyklisches Carbonat-Lösungsmittel umfasst.

10. Elektrolyt für eine Lithium-Sekundärbatterie nach einem der Ansprüche 1 bis 9, außerdem umfassend mindestens ein Hilfsadditiv, ausgewählt aus der Gruppe bestehend aus einer ungesättigten cyclischen Carbonatverbindung, einer fluorsubstituierten Carbonatverbindung, einer Sultonverbindung, einer cyclischen Sulfatverbindung und einer Phosphatverbindung.

11. Elektrolyt für eine Lithium-Sekundärbatterie nach Anspruch 10, wobei der Gehalt des Hilfsadditivs 0,05 bis 10 Gew.-%, bezogen auf das Gesamtgewicht des Elektrolyten für eine Lithium-Sekundärbatterie, beträgt.

12. Elektrolyt für eine Lithium-Sekundärbatterie nach einem der Ansprüche 10 oder 11, wobei das Verhältnis des Gehalts an dem Hilfsadditiv zum Gehalt an dem Additiv 0,1 bis 10, bezogen auf das Gewicht, beträgt.

13. Lithium-Sekundärbatterie, umfassend:
eine Kathode;
eine Anode, die so angeordnet ist, dass sie der Kathode gegenüberliegt; und
den Elektrolyten für eine Lithium-Sekundärbatterie nach einem der Ansprüche 1 bis 12, in dem die Kathode und die Anode imprägniert sind.

## Revendications

1. Électrolyte pour une batterie secondaire au lithium, comprenant :
un additif comprenant un composé représenté par la formule 1 ci-dessous ou un composé représenté par la formule 2 ci-dessous ;
un solvant organique ; et
un sel de lithium : 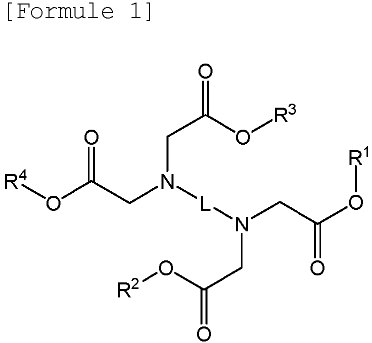 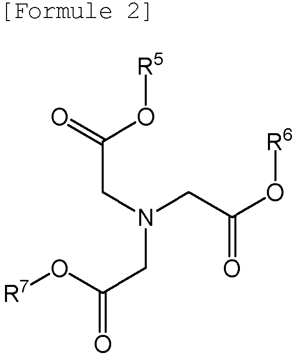
(dans la formule 1 et la formule 2 ci-dessus s'applique,
R¹ à R⁷ sont chacun indépendamment un groupe alkyle substitué ou non de 1 à 10 atomes de carbone, et L est un groupe alkylène substitué ou non de 1 à 6 atomes de carbone).

2. Électrolyte pour une batterie secondaire au lithium selon la revendication 1, dans lequel l'additif comprend un composé représenté par la formule 1-1 ci-dessous : 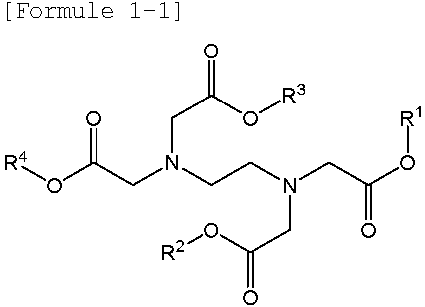 (dans la formule 1-1 ci-dessus, R¹ à R⁴ sont chacun indépendamment un groupe alkyle substitué ou non substitué ayant de 1 à 10 atomes de carbone).

3. Électrolyte pour une batterie secondaire au lithium selon la revendication 1 ou 2, dans lequel R¹ à R⁷ dans les formules 1 et 2 ci-dessus sont chacun indépendamment un groupe alkyle non substitué ayant 1 à 3 atomes de carbone.

4. Électrolyte pour une batterie secondaire au lithium selon l'une quelconque des revendications 1 à 3, dans laquelle R¹ à R⁷ dans les formules 1 et 2 ci-dessus sont un groupe méthyle.

5. Électrolyte pour une batterie secondaire au lithium selon l'une quelconque des revendications 1 à 4, dans lequel l'additif comprend un composé de formule 1-2 ci-dessous ou un composé de formule 2-1 ci-dessous :

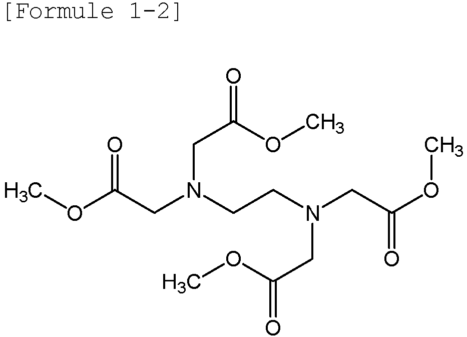

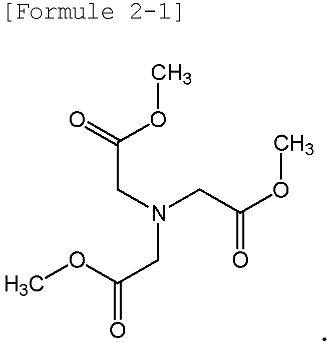

6. Électrolyte pour une batterie secondaire au lithium selon l'une quelconque des revendications 1 à 5, dans lequel la teneur en l'additif est de 0,1 à 5 % en poids, par rapport au poids total de l'électrolyte pour une batterie secondaire au lithium.

7. Électrolyte pour une batterie secondaire au lithium selon l'une quelconque des revendications 1 à 6, dans lequel l'additif comprend le composé représenté par la formule 1 ci-dessus et le composé représenté par la formule 2 ci-dessus ensemble.

8. Électrolyte pour une batterie secondaire au lithium selon la revendication 7, dans lequel le rapport de la teneur du composé représenté par la formule 2 ci-dessus à la teneur du composé représenté par la formule 1 ci-dessus est de 0,25 à 4 en poids.

9. Électrolyte pour une batterie secondaire au lithium selon l'une quelconque des revendications 1 à 8, dans lequel le solvant organique comprend un solvant carbonate linéaire et un solvant carbonate cyclique.

10. Électrolyte pour une batterie secondaire au lithium selon l'une quelconque des revendications 1 à 9, comprenant en outre au moins un additif auxiliaire choisi dans le groupe constitué par un composé carbonate cyclique insaturé, un composé carbonate substitué par du fluor, un composé sultone, un composé sulfate cyclique et un composé phosphate.

11. Électrolyte pour une batterie secondaire au lithium selon la revendication 10, dans lequel la teneur de l'additif auxiliaire est de 0,05 à 10 % en poids, par rapport au poids total de l'électrolyte pour une batterie secondaire au lithium.

12. Électrolyte pour une batterie secondaire au lithium selon l'une des revendications 10 ou 11, dans lequel le rapport de la teneur en l'additif auxiliaire à la teneur en l'additif est de 0,1 à 10 en poids.

13. Batterie secondaire au lithium, comprenant
une cathode ;
une anode disposée de manière à faire face à la cathode ; et
l'électrolyte pour une batterie secondaire au lithium selon l'une quelconque des revendications 1 à 12, dans lequel la cathode et l'anode sont imprégnées.
